# EUROPÄISCHE PATENTSCHRIFT

(11) **EP 0 331 983 B1**
(45) Veröffentlichungstag und Bekanntmachung des Hinweises auf die Patenterteilung: **14.12.1994**
(21) Anmeldenummer: 89103182.5
(22) Anmeldetag: 23.02.1989
(51) Int. Cl.: C07D 295/08, C07C 323/16, C07C 317/22, C07C 317/32, C07C 323/32, C07C 211/53, C07D 295/12, A61K 31/33, A61K 31/135, A61K 31/10, C07C 223/06

(54) **Neue Stilbenderivate, ihre Herstellung und Verwendung für Heilmittel**
Stilbene derivatives, their preparation and their use in medicines
Dérivés du stilbène, leur préparation et leur utilisation dans les médicaments

(30) Priorität: 24.02.1988 CH 689/88; 14.12.1988 CH 4622/88
(43) Veröffentlichungstag der Anmeldung: 13.09.1989
(73) Patentinhaber: F. HOFFMANN-LA ROCHE AG, 4002 Basel (CH)
(72) Erfinder: Klaus, Michael, Dr., D-7858 Weil/Rhein (DE); Mohr, Peter, Dr., CH-4051 Basel (CH); Weiss, Ekkehard, Dr., D-7854 Inzlingen (DE)
(74) Vertreter: Lederer, Franz, Dr.

(56) Entgegenhaltungen:
- EP-A- 0 004 066
- EP-A- 0 029 412
- EP-A- 0 051 187
- EP-A- 0 058 370
- BE-A- 641 160
- CH-A- 502 301
- DE-A- 1 645 581
- DE-C- 544 087
- FR-A- 2 599 027
- GB-A- 456 534
- GB-A- 1 208 980
- US-A- 3 483 209
- CHEMICAL ABSTRACTS, Band 76, 22. Mai 1972, Seite 527, Zusammenfassung Nr.127329p, Columbus, Ohio, US; A.M. BELIKOVA et al.: "Alkylation of nucleic acidsand their components. IV. Alkylation of nucleosides by 4-(N-beta-chloroethyl-N-methyl-amino)benzaldehyde",& KHIM.GETEROTSIKL. SOEDIN. 1972, (1), 109-16
- CHEMICAL ABSTRACTS, Band 87, 5. September 1977, Seite 75, Zusammenfassung Nr.69720y, Columbus, Ohio, US; A.T. PETERS et al.: "Styryl for synthetic-polymerfibers. Part I. Synthesis and characterization of some 4-amino-beta,beta-dicyanostyrenes and ethyl-alpha-cyano-4-aminocinnamates",& J.SOC. DYERS COLOUR. 1977, 93(4), 126-33
- CHEMICAL ABSTRACTS, Band 107, 7. September 1987, Seite 610, Zusammenfassung Nr.87107q, Columbus, Ohio, US; & JP-A-62 00 964 (MINOLTA CAMERA CO., LTD) 06-01-1987
- CHEMICAL & PHARMACEUTICAL BULLETIN,Band 31, Nr. 6, Juni 1983, Seiten 2023-2032; S. NARUTO et al.: "Synthesis andspasmolytic activity of 2-substituted-3-(omega-dialkylamino-alkoxyphenyl)acrylonitriles and related compounds"

## Beschreibung

Die vorliegende Erfindung betrifft neue Stilbenderivate der allgemeinen Formel 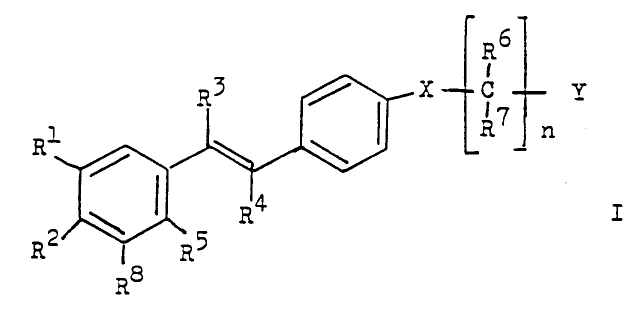
worin R¹ und R² unabhängig voneinander nieder Alkyl; oder zusammengenommen 1,3-Propylen, 1,4-Butylen und 1,5-Pentylen und nieder-Alkyl-substituierte Derivate davon; einer der Reste R³ und R⁴ Wasserstoff und der andere Wasserstoff oder nieder-Alkyl; R⁶ und R⁷ Wasserstoff oder nieder-Alkyl; R⁵ und R⁸ Wasserstoff, nieder-Alkyl, nieder-Alkoxy oder Halogen; X -O-; Y Piperidino, Pyrrolidino, Morpholino, Piperazino, N-Methylpiperazino, Thiomorpholino, Thiomorpholino-4-oxid, Thiomorpholino-4,4-dioxid, Imidazolino oder Pyrrolo und n 2, 3 oder 4 bedeutet.

Die Erfindung betrifft weiterhin ein Verfahren zur Herstellung der Verbindungen der Formel I, pharmazeutische Präparate auf Basis der Verbindungen der Formel I, die Verbindungen der Formel I bei der Behandlung und Prophylaxe von Neoplasien, Dermatosen und Altershaut sowie der Verwendung der Verbindungen der Formel I bei der Herstellung von pharmazeutischen Präparaten zur Behandlung und Prophylaxe solcher Erkrankungen.

Die Bezeichnung "nieder" bezeichnet Gruppen mit 1-6 C-Atomen. Alkyl- und Alkoxygruppen können geradkettig oder verzweigt sein, wie Methyl, Aethyl, Propyl, Isopropyl, Butyl, sek,-Butyl oder tert.-Butyl bzw. Methoxy, Aethoxy, Propoxy, Isopropoxy, Butoxy, sek.-Butoxy und tert.-Butoxy. Halogen umfasst Fluor, Chlor, Brom und Jod. Ein durch R¹ und R² gemeinsam dargestellter Alkylrest mit 3-5 C-Atomen in gerader Kette kann Verzweigungen aufweisen, Beispiele für nieder-Alkyl-substituierte Derivate von 1,3-Propylen, 1,4-Butylen und 1,5-Pentylen sind die Reste -C(CH₃)₂-CH₂-C(CH₃)₂-, -C(CH₃)₂-CH₂CH₂-C(CH₃)2-und -CH₂CH₂-C(CH₃)₂-CH₂CH₂-.

Die Verbindungen der Formel I können als trans- oder cis-Isomere oder cis/trans- Isomerengemische vorliegen. Im allgemeinen sind die trans-Verbindungen der Formel I bevorzugt.

Bevorzugte Gruppen von Verbindungen der Formel I sind solche, in denen R¹ und R² gemeinsam einen Rest -C(CH₃)₂-CH₂-C(CH₃)₂- oder -C(CH₃)₂-CH₂CH₂-C(CH₃)₂-darstellen, R⁴, R⁵ und R⁸ Wasserstoff; und R³ Methyl darstellen. Weiterhin sind die Verbindungen der Formel I, in denen R¹ und R⁸ nieder-Alkyl, insbesondere tert.-Butyl, sind, von besonderem Interesse. Ferner ist das 4-[2-[p-[(E)-2-(5,6,7,8-Tetrahydro-5,5,8,8-tetramethyl -2-naphthyl)propenyl]phenoxy]äthyl]morpholin bevorzugt.

Typische Vertreter der erfindungsgemässen Verbindungen sind die in den Beispielen beschriebenen sowie die nach stehend aufgeführten Verbindungen:
1-Methyl-4-[2-[p-[(E)-2-(5,6,7,8-tetrahydro-5,5,8,8-tetramethyl-2-naphthyl)propenyl]phenoxy]äthyl]piperazin;
Tetrahydro-4-[2-[p-[(E)-2-(5,6,7,8-tetrahydro-5,5,8,8-tetramethyl-2-naphthyl)propenyl]phenoxy]äthyl]-2H-1,4-thiazin;
1-[2-[p-[(E)-2-(5,6,7,8-Tetrahydro-5,5,8,8-tetramethyl-2-naphthyl]propenyl]phenoxy]äthyl]pyrrol;
Die Verbindungen der Formel I können erfindungsgemäss dadurch erhalten werden, dass man
a) eine Verbindung der allgemeinen Formel 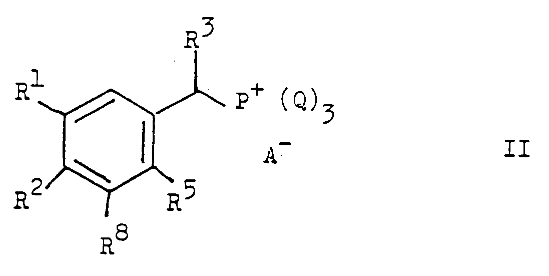 mit einer Verbindung der allgemeinen Formel 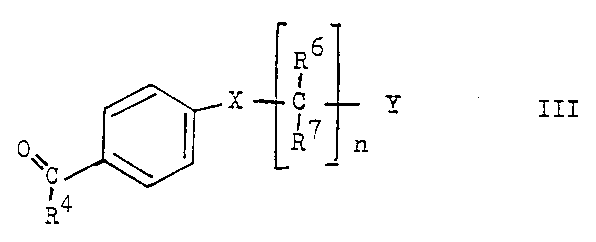 oder
b) eine Verbindung der allgemeinen Formel 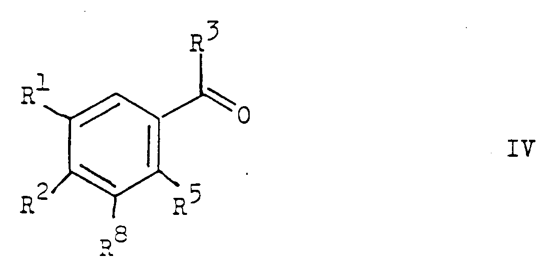 mit einer Verbindung der allgemeinen Formel 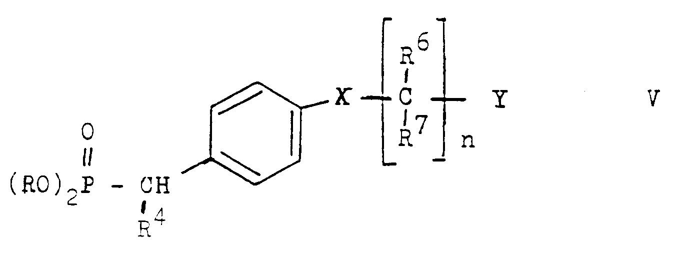 oder
c) eine Verbindung der allgemeinen Formel 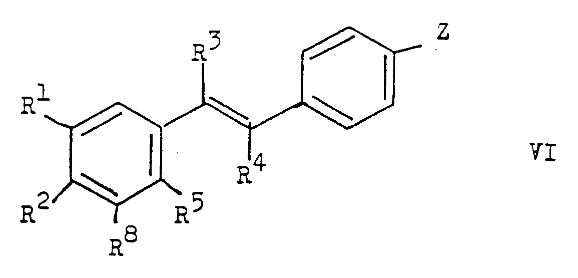 mit einer Verbindung der allgemeinen Formel 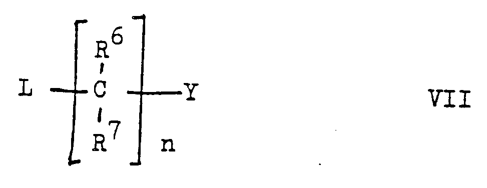 umsetzt wobei in den vorgenannten Formeln II-VII R¹, R², R³, R⁴, R⁵, R⁶, R⁷, R⁸, X, Y und n die oben angegebene Bedeutung haben; Q Aryl; A⁻ ein Anion einer anorganischen oder organischen Säure; R eine niedere Alkylgruppe; Z Hydroxy, und L eine Abgangsgruppe darstellen.

Die Umsetzung gemäss Verfahrensvariante a) kann unter den üblichen Verfahrensbedingungen einer Wittig-Reaktion durchgeführt werden. Dabei werden die Komponenten in Gegenwart eines säurebindenden Mittels, z.B. in Gegenwart einer starken Base, wie z.B. Butyllithium, Natriumhydrid, Kalium-tert.-butylat oder dem Natriumsalz von Dimethylsulfoxyd, vornehmlich aber in Gegenwart eines gegebenenfalls durch niederes Alkyl substituierten Aethylenoxyds, wie 1,2-Butylenoxyd, gegebenenfalls in einem Lösungsmittel, z.B. in einem Aether, wie Diäthyläther oder Tetrahydrofuran, oder in einem aromatischen Kohlenwasserstoff, wie Benzol oder Toluol, in einem zwischen Raumtemperatur und dem Siedepunkt des Reaktionsgemisches liegenden Temperaturbereich miteinander umgesetzt.

Von den anorganischen Säureanionen A⁻ ist das Chlor- und Brom-ion oder das Hydrosulfat-ion, von den organischen Säureanionen ist das Tosyloxy-ion bevorzugt. Der Arylrest Q ist vorzugsweise ein Phenylrest oder ein substituierter Phenylrest, wie p-Tolyl.

Die Umsetzung nach Verfahrensvariante b) kann unter den üblichen Bedingungen einer Horner-Reaktion durchgeführt werden. Dabei werden die Komponenten mit Hilfe einer Base und vorzugsweise in Gegenwart eines inerten organischen Lösungsmittels, z.B. mit Hilfe von Natriumhydrid in Benzol, Toluol, Dimethylformamid, DMSO. Tetrahydrofuran, Dioxan oder 1,2-Dimethoxyäthan, oder auch mit Hilfe eines Natriumalkoholates in einem Alkanol, z.B. Natriummethylat in Methanol, in einem zwischen 0° und dem Siedepunkt des Reaktionsgemisches liegenden Temperaturbereich kondensiert.

Die Alkoxyreste RO sind vornehmlich niedere Alkoxyreste mit 1-6 Kohlenstoffatomen, wie Methoxy oder Aethoxy.

Die Umsetzung nach Verfahrensvariante c) kann in einem organischen Lösungsmittel wie Dimethylformamid, zweckmässig unter Erwärmen bis zur Rückflusstemperatur des Reaktionsgemisches, vorgenommen werden. Beispiele von Abgangsgruppen L sind Halogen, wie Chlor; Mesyloxy und Tosyloxy.

Die Ausgangsverbindungen der allgemeinen Formeln II-VII können, soweit sie nicht bekannt oder hier beschrieben sind, in Analogie zu bekannten oder hier beschriebenen Methoden hergestellt werden.

Verbindungen der allgemeinen Formel II und IV und ihre Herstellung sind z.B. in den deutschen Offenlegungs Schriften 2 414 619 und 2 819 213 und der europäischen Patentschrift 2 742 beschrieben.

Verbindungen der allgemeinen Formel III können ausgehend von p-Hydroxy- substituierten Benzaldehyd oder Acetophenon, Propiophenon oder Homologen davon durch Umsetzung mit einer Verbindung der Formel Y(CR⁶,R⁷)ₙCl in Gegenwart einer Base, wie NaH hergestellt werden.

Verbindungen der allgemeinen Formel V können ausgehend von p-Hydroxy-substituierten Benzoesäureestern hergestellt werden durch Umsetzung mit einer Verbindung der Formel Y(CR⁶,R⁷)ₙCl. Reduktion der Estergruppe mit Dibal oder LiAlH₄ zum entsprechenden Alkohol, Austausch der Hydroxygruppe gegen Brom z.B. durch Umsetzung mit PBr₃ und Umsetzung des Bromids mit Trialkylphosphit zum Phosphonat der allgemeinen Formel V.

Verbindungen der allgemeinen Formel VI, in denen Z Hydroxy darstellt, können aus Verbindungen der allgemeinen Formel II und Verbindungen der allgemeinen Formel 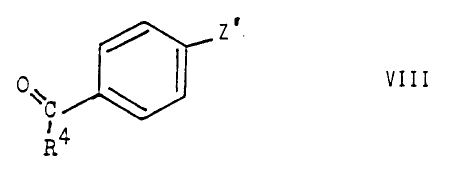
wobei Z' eine geschützte Hydroxygruppe, z.B. eine Acetoxygruppe ist,
durch Wittig-Reaktion und anschliessende Abspaltung der Schutzgruppe hergestellt werden. Die Dokumente EP-A-58 370 und FR-A-2 599 027 beschreiben Arotinoide mit ähnlichem Wirkungsspektrum wie die vorliegenden Verbindungen, die jedoch im terminalen Phenylring Alkoxy- oder Hydroxyalkylsulfonyl-gruppen enthalten können. Das Dokument EP-A-51 187 beschreibt Styrylfarbstoffe, die Dimethylamino-äthylaminogruppen als Substituenten an einem Phenylring enthalten. Das Dokument US-A-3 483 209 beschreibt Bis-(Phenylalkylaminoalkyl)benzolderivate, die in den endständigen Phenylringen durch Pyrrolidinoäthoxygruppen substituiert sein können und die antibakteriell und anti-parasitisch wirksam sind. Das Dokument Chem.Pharm. Bull.,31(6) 2023-2032 (1983) beschreibt spasmolytisch wirksame Benzisoxazolyl-phenyl-acrylnitrile, die im Phenylring durch Morpholino- oder Piperidino-äthoxy substituiert sind.

Die biologische Wirksamkeit der erfindungsgemässen Verbindung kann z.B. mit den in der deutschen Offenlegungsschrift 3 715 955 beschriebenen Versuchsanord- nungen ermittelt werden. Bei der Prüfung der Wirkung gegen chemisch induzierte (orale Verabreichung von Dimethylbenzanthracen) Mammatumoren bei Ratten wurden mit der Verbindung der Formel I, 4-[2-[p-[(E)-2-(5,6,7,8-Tetrahydro-5,5,8,8-tetramethyl -2-naphthyl)propenyl]phenoxy]äthyl]morpholin die nachstehend zusammengefassten Resultate erhalten:

| | Dosis mg/kg/Tag] p.o. | Ratten mit Tumoren [% von Kontrollen] | Zunahme oder Abnahme der durchschnittlichen Anzahl von Tumoren pro Ratte | Zunahme oder Abnahme des durchschnittlichen Tumorvolumens pro Ratte in mm³ |
|---|---|---|---|---|
| | 50 | 100 | +91 | +550 |
| | 100 | 50 | -73 | - 77 |
| | 200 | 10 | -96 | - 99 |
| Kontrollgruppe | - | 100 | +242 | +714 |

Die Verbindungen der Formel I können zur topischen und systemischen Therapie von benignen und malignen Neoplasien, von prämalignen Läsionen, sowie ferner auch zur systemischen und topischen Prophylaxe der genannten Affektionen verwendet werden.

Sie sind des weiteren für die topische und systemische Therapie von Akne, Psoriasis und anderen, mit einer verstärkten oder pathologisch veränderten Verhornung einhergehenden. Dermatosen, wie auch von entzündlichen und allergischen dermatologischen Affektionen geeignet. Die Verfahrensprodukte der Formel I können ferner auch zur Bekämpfung von Schleimhauterkrankungen mit entzündlichen oder degenerativen bzw. metaplastischen Veränderungen eingesetzt werden. Weiterhin können die Verbindungen der Formel I, vorzugsweise in topischen Präparaten, zur Behandlung und Prophylaxe lichtgeschädigter Haut (Altershaut) verwendet werden.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für die parenterale Applikation sind Mittel in Form von Infusions- oder Injektionslösungen geeignet.

Die Dosierungen, in denen die Präparate verabreicht werden, können je nach Anwendungsart und Anwendungsweg sowie nach den Bedürfnissen der Patienten variieren. Im allgemeinen kommen für den Erwachsenen tägliche Dosen von etwa 0,1-100 mg/kg, vorzugsweise 1-50 mg/kg in Betracht.

Die Präparate können inerte oder auch pharmakodynamisch aktive Zusätze enthalten. Tabletten oder Granula z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dgl. bestehen. Voraussetzung ist, dass alle bei der Herstellung der Präparate verwendeten Hilfsstoffe untoxisch sind.

Zur topischen Anwendung werden die Wirkstoffe zweckmässig in Form von Salben, Tinkturen, Crèmen, Lösungen, Lotionen, Sprays, Suspensionen und dgl. verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen, festen oder flüssigen Trägern zumischt.

Für die topische Anwendung sind zweckmässig ca. 0,1-5%ige, vorzugsweise 0,3-2%ige Lösungen, sowie ca. 0,1-5%ige, vorzugsweise ca. 0,3-2%ige Salben oder Crèmen geeignet.

Den Präparaten kann gegebenenfalls ein Antioxydationsmittel, z.B. Tocopherol, N-Methyl-q-tocopheramin sowie butyliertes Hydroxyanisol oder butyliertes Hydroxytoluol beigemischt sein.

Die nachstehenden Beispiele erläutern die Erfindung weiter. Die Temperaturen sind in Celsiusgraden angegeben.

### Beispiel 1

332 g [1-(5,6,7,8-Tetrahydro-5,5,8,8-tetramethyl -2-naphthyl)äthyl]triphenylphosphoniumbromid werden in 1,2 l Tetrahydrofuran suspendiert. Unter Rühren tropft man bei 0°C 406 ml Butyllithium (1,6 molar in Hexan) hinzu. Nach 1-stündigem Rühren bei 0°C tropft man zu der dunkelroten Lösung eine Lösung von 120 g 4-(2-Morpholinoäthoxy)benzaldehyd in 400 ml Tetrahydrofuran. Nach 2-stündigem Rühren bei Raumtemperatur giesst man das Reaktionsgemisch in 3 l eines Methanol/Wasser-Gemisches (6:4) und extrahiert mit Hexan. Die organische Phase wird mit Wasser gewaschen, über Natriumsulfat getrocknet und eingedampft. Der gelbliche, kristalline Rückstand wird aus Essigester/Hexan umkristallisiert und ergibt 123 g 4-[2-[p-[(E)-2-(5,6,7,8-Tetrahydro-5,5,8,8-tetramethyl-2-naphthyl)propenyl]phenoxy]äthyl]-morpholin in weissen Kristallen, Schmelzpunkt 107-109°C.

Der als Ausgangsprodukt verwendete 4-(2-Morpholinoäthoxy)benzaldehyd kann wie folgt hergestellt werden:

92,8 g 4-Hydroxybenzaldehyd werden in 820 ml Dimethylformamid gelöst. Nach Zugabe von 228 g 4-(2-Chloräthyl)morpholin und 415 g fein gepulvertem Kaliumcarbonat wird das Reaktionsgemisch unter Argon und kräftigem Rühren über Nacht auf 100°C erwärmt. Die abgekühlte Lösung wird auf 3 l Eiswasser gegossen, mit Essigester extrahiert, mit Wasser gewaschen, getrocknet und eingedampft Das zurückbleibende dunkle Oel wird am Hochvakuum destilliert und ergibt 132 g 4-(2-Morpholinoäthoxy)benzaldehyd als gelbliches Oel, Siedepunkt 145-150°C/33 Pa.

### Beispiel 2

In zu Beispiel 1 analoger Weise erhält man aus 26 g Triphenyl [1-(1,1,3,3-tetramethyl-5-indenyl)äthyl]phosphoniumbromid und 10 g 4-(2-Morpholinoäthoxy)benzaldehyd nach Umkristallisation aus Hexan 13,6 g 4-[2-[p-[(E)-2-(1,1,3,3-Tetramethyl -5-indenyl)propenyl]phenoxy]äthyl]morpholin in weissen Kristallen, Schmelzpunkt 85-86°C.

### Beispiel 3

0,75 g einer 50%igen Suspension von Natriumhydrid in Mineralöl werden in 10 ml Dimethylformamid suspendiert. Unter Rühren tropft man bei 0°C eine Lösung von 5 g p-[(E)-2-(5,6,7,8-Tetrahydro- 5,5,8,8- tetramethyl -2-naphthyl)-propenyl]phenol in 30 ml Dimethylformamid hinzu. Nach 1-stündigem Rühren bei 0°C wird eine Lösung von 3,6 g N-(2-Chloräthyl)pyrrolidin in 30 ml Dimethylformamid tropfenweise hinzugefügt. Das Reaktionsgemisch wird anschliessend 2 Stunden auf 70°C erwärmt, danach abgekühlt, auf Eis-Wasser gegossen und mit Essigester extrahiert. Die organische Lösung wird mehrfach mit Wasser gewaschen, getrocknet und eingedampft Man erhält ein gelbbraunes Oel, das durch Filtration über Kieselgel (Eluierungsmittel Essigester + 10% Aethanol) gereinigt und aus Hexan umkristallisiert wird. Isoliert werden 4,5 g 1-[2-[p-[(E)-2-(5,6,7,8,-Tetrahydro-5,5,8,8-tetramethyl -2-naphthyl)propenyl]phenoxy]äthyl]pyrrolidin in weissen Kristallen, Schmelzpunkt 80-82°C.

### Beispiel 4

In zu Beispiel 3 analoger Weise erhält man aus 5 g p-[(E)-2-(5,6,7,8-Tetrahydro-5,5,8,8-tetramethyl -2-naphthyl)propenyl]phenol und 4,7 g 1-(2-Chloräthyl)-piperidin 5,2 g 1-[2-[p-[(E)-2-(5,6,7,8-Tetrahydro-5,5,8,8-tetramethyl -2-naphthyl)propenyl]phenoxy]äthyl]piperidin in weissen Kristallen, Schmelzpunkt 91-93°C.

### Beispiel 5

2,1 g [1-(6,7,8,9-Tetrahydro-7,7-dimethyl-5H-benzocyclohepten -2-yl)äthyl]triphenylphosphoniumbromid werden in 10 ml Toluol suspendiert und bei 0°C mit 0,5 g Kalium-tert-butylat versetzt. Nach 2-stündigem Rühren bei Raumtemperatur tropft man eine Lösung von 0,94 g 4-(2-Morpholinoäthoxy)benzaldehyd in 5 ml Toluol hinzu und rührt 3 Stunden bei Raumtemperatur nach. Nach dem Abdampfen der Hauptmenge des Lösungsmittels giesst man auf ein Gemisch aus Methanol/Wasser (6:4) und extrahiert mehrfach mit Hexan. Die organische Phase wird mit Wasser gewaschen, getrocknet und eingedampft. Umkristallisation des Rückstandes aus Hexan ergibt 0,4 g 4-[2-[p-[(E)-2-(6,7,8,9-Tetrahydro-7,7-dimethyl -5H-benzocyclohepten-2-yl)propenyl]phenoxy]äthyl]morpholin, Schmelzpunkt 78-79°C.

### Beispiel 6

Analog Beispiel 1 erhält man aus [1-(5,6,7,8-Tetrahydro-5,5,8,8-tetramethyl-2-paphthyl)äthyl]triphenylphosphoniumbromid und 4-[2-(Tetrahydro-4'H-1,4-thiazin-4'-yl)äthoxy]benzaldehyd 1',1'-dioxid nach 16-stündigem Rühren bei Raumtemperatur, Aufarbeitung, Flashchromatographie an Kieselgel mit Hexan-Essigester (1:2) und Kristallisation aus Essigester-Hexan das Tetrahydro 4-[2-[p-[(E)-2-(5,6,7,8-tetrahydro-5,5,8,8-tetramethyl-2-naphthyl)propenyl]phenoxy]äthyl]-2H-1,4-thiazin 1,1-dioxid vom Schmelzpunkt 144-145°.

Das 4-[2-(Tetrahydro-4'H-1,4-thiazin-4'-yl)äthoxy]benzaldehyd 1',1'-dioxid kann wie folgt hergestellt werden:

11,6 g 4-Hydroxybenzaldehyd in 160 ml Dimethylformamid werden mit 15,6 g 4-(2-Chloräthyl)-tetrahydro-2H-1,4-thiazin 1,1-dioxid und 21,6 g pulverisiertem Kaliumcarbonat unter Argon 2 Stunden auf 100° erwärmt. Nach dem Abkühlen wird auf 150 g Eis gegossen, dreimal mit 300 ml Essigester extrahiert und die organischen Phasen noch zweimal mit 150 ml Wasser gewaschen, über Natriumsulfat getrocknet und im Vakuum eingedampft. Der beige, kristalline Rückstand (22,1 g) wird direkt für obige Wittigreaktion verwendet. Eine Probe wurde an Kieselgel mit Essigester-Hexan (4:1) chromatographiert und lieferte aus Essigester-Hexan farblose Kristalle vom Schmelzpunkt 101-102°C.

Die Herstellung von Gebrauchsformen der Verbindungen der Formel I kann in üblicher Weise, z.B. anhand der nachstehenden Beispiele, erfolgen.

### Beispiel A

Hartgelatinekapseln können wie folgt hergestellt werden:

| Bestandteile | mg/Kapsel |
|---|---|
| 1. sprühgetrocknetes Pulver, enthaltend 75% Verbindung I | 200 |
| 2. Natriumdioctylsulfosuccinat | 0,2 |
| 3. Natriumcarboxymethylcellulose | 4,8 |
| 4. mikrokristalline Cellulose | 86,0 |
| 5. Talk | 8,0 |
| 6. Magnesiumstearat | 1,0 |
| | Total 3̅0̅0̅ |

Das sprühgetrocknete Pulver, das auf dem Wirkstoff, Gelatine und mikrokristalliner Cellulose basiert und eine mittlere Korngrösse des Wirkstoffes von <1 m aufweist (mittels Autokorrelationsspektroskopie gemessen), wird mit einer wässrigen Lösung von Natriumcarboxymethylcellulose und Natriumdioctylsulfosuccinat befeuchtet und geknetet. Die resultierende Masse wird granuliert, getrocknet und gesiebt, und das erhaltene Granulat mit mikrokristalliner Cellulose, Talk und Magnesiumstearat vermischt. Das Pulver wird in Kapseln der Grösse 0 abgefüllt.

### Beispiel B

Tabletten können wie folgt hergestellt werden:

| Bestandteile | mg/Tablette |
|---|---|
| 1. Verbindung 1 als feingemahlenes Pulver | 500 |
| 2. Milchzucker pulv. | 100 |
| 3. Maisstärke weiss | 60 |
| 4. Povidone® K30 | 8 |
| 5. Maisstärke weiss | 112 |
| 6. Talk | 16 |
| 7. Magnesiumstearat | 4 |
| | Total 8̅0̅0̅ |

Die feingemahlene Substanz wird mit Milchzucker und einem Teil der Maisstärke gemischt. Die Mischung wird mit einer wässrigen Lösung von Povidone® K30 befeuchtet und geknetet, und die resultierende Masse granuliert, getrocknet und gesiebt. Das Granulat wird mit der restlichen Maisstärke. Talk und Magnesiumstearat vermischt und zu Tabletten geeigneter Grösse verpresst.

### Beispiel C

Weichgelatinekapseln können wie folgt hergestellt werden:

| Bestandteile | mg/Kapsel |
|---|---|
| 1. Verbindung I | 50 |
| 2. Triglycerid | 450 |
| | Total 5̅0̅0̅ |

10 g Verbindung I werden Unter Rühren, Inertbegasung und Lichtschutz in 90 g mittelkettigem Triglycerid gelöst. Diese Lösung wird als Kapselfüllmasse zu Weichgelatinekapseln à 50 mg Wirkstoff verarbeitet.

### Beispiel D

Eine Lotion kann wie folgt hergestellt werden:

| Bestandteile | |
|---|---|
| 1. Verbindung I, feingemahlen | 3,0 g |
| 2. Carbopol® 934 | 9,6 g |
| 3. Natriumhydroxid | q.s. ad pH 6 |
| 4. Aethanol. 94% | 50,0 g |
| 5. entmineralisiertes Wasser | ad 100,0 g |

Der Wirkstoff wird unter Lichtschutz in die Mischung Aethanol, 94%ig/Wasser eingearbeitet. Carbopol® 934 wird bis zur vollständigen Gelierung eingerührt und der pH-Wert mit Natriumhydroxid eingestellt.

## Patentansprüche (Patentansprüche für folgende(n) Vertragsstaat(en): AT, BE, CH, DE, FR, GB, IT, LI, LU, NL, SE)

1. Verbindungen der allgemeinen Formel 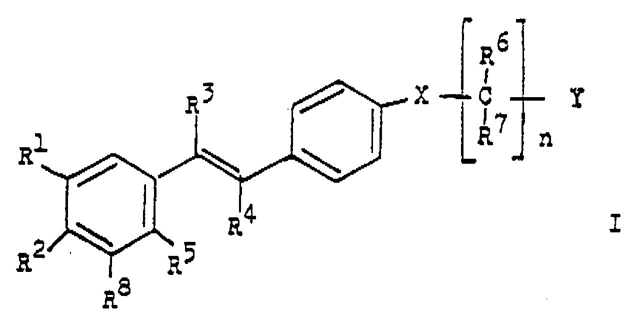 worin R¹ und R² unabhängig voneinander nieder Alkyl; oder zusammengenommen 1,3-Propylen, 1,4-Butylen und 1,5-Pentylen und nieder-Alkyl-substituierte Derivate davon; einer der Rente R³ und R⁴ Wasserstoff und der andere Wasserstoff oder nieder-Alkyl; R⁶ und R⁷ Wasserstoff oder nieder-Alkyl; R⁵ und R⁸ Wasserstoff, nieder-Alkyl, nieder-Alkoxy oder Halogen; X -O-; Y Piperidino, Pyrrolidino, Morpholino, Piperazino, N-Methylpiperazino, Thiomoryholino, Thiomorpholino-4-oxid, Thiomorpholino-4,4-dioxid, Imidazolino oder Pyrrolo und n 2, 3 oder 4 bedeutet und wobei "nieder" Gruppen mit 1-6 C - Atomen bezeichnet.

2. Verbindungen gemäss Anspruch 1, in denen R¹ und R² gemeinsam einen Rest -(CH₃)₂C-CH₂CH₂-C(CH₃)₂- oder -(CH₃)₂C-CH₂-C(CH₃)₂- darstellen.

3. 4-[2-[p-[(E)-2-(5,6,7,8-Tetrahydro-5,5,8,8-tetramethyl-2-naphthyl)propenyl]phenoxy]äthyl]morpholin als Verbindung gemäss Anspruch 1.

4. 4-[2-[p-[(E)-2-(1,1,3,3-Tetramethyl-5-indenyl)propenyl]phenoxy]äthyl]morpholin.
1-[2-[p-(E)-2-(5,6,7,8-Tetrahydro-5,5,8,8-tetramethyl-2-naphthyl)propenyl]phenoxy]äthyl]pyrrolidin,
1-[2-[p-[(E)-2-(5,6,7,8-Tetrahydro-5,5,8,8-tetramethyl-2-naphthyl)propenyl]phenoxy]äthyl]piperidin,
4-[2-[P-[(E)-2-(6,7,8,9-Tetrahydro-7,7-dimethyl-5H-benzocyclohepten-2-yl)propenyl]phenoxy]äthyl]morpholin,
Tetrahydro-4-[2-[p-[(E)-2-(5,6,7,8-tetrahydro-5,5,8,8-tetrametmethyl-2-naphthyl)propenyl]phenoxy]äthyl]-2H-1,4-thiazin-1,1-dioxid,
als Verbindungen gemäss Anspruch 1.

5. Die Verbindungen gemäss den Ansprüchen 1-4 zur Verwendung als Heilmittel.

6. Verfahren zur Herstellung der Verbindungen der allgemeinen Formel I von Anspruch 1, dadurch gekennzeichnet, dass man
a) eine Verbindung der allgemeinen Formel 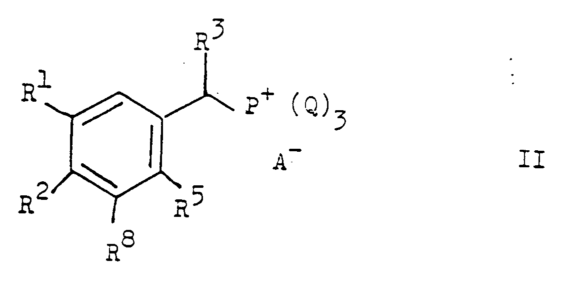 mit einer Verbindung der allgemeinen Formel 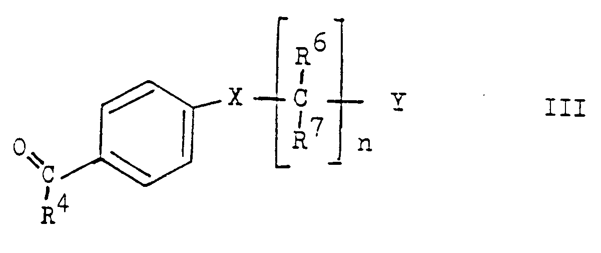 oder
b) eine Verbindung der allgemeinen Formel 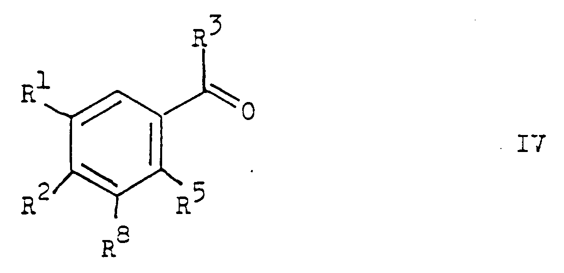 mit einer Verbindung der allgemeinen Formel 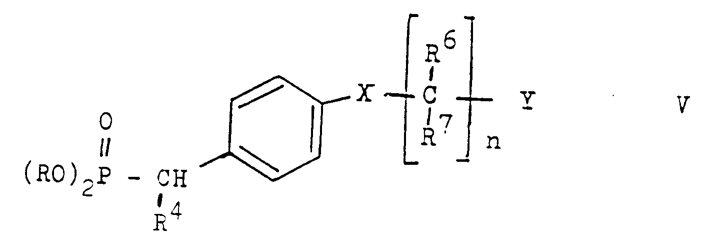 oder
c) eine Verbindung der allgemeinen Formel 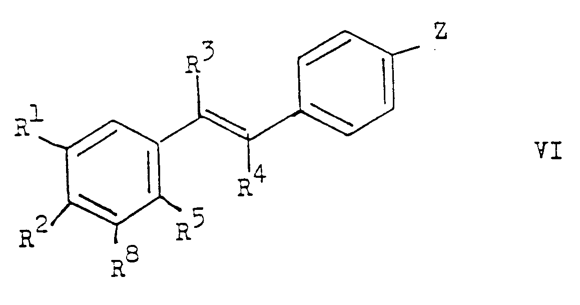 mit einer Verbindung der allgemeinen Formel 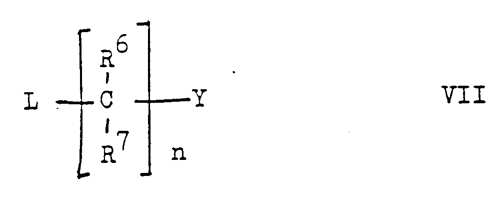 umsetzt wobei in den vorgenannten Formeln II-VII R¹, R², R³, R⁴, R⁵, R⁶, R⁷, R⁸, X, Y und n die oben angegebene Bedeutung haben; Q Aryl: A⁻ ein Anion einer anorganischen oder organischen Säure; R eine niedere Alkylgruppe; Z Hydroxy; und L eine Abgangsgruppe darstellen.

7. Pharmazeutische Präparate, enthaltend eine Verbindung gemäss den Ansprüchen 1-4 und übliche pharmazeutische Hilfs- und Trägerstoffe.

8. Verwendung der Verbindungen gemäss den Ansprüchen 1-4 als Wirkstoffe bei der Herstellung pharmazeutischer Präparate, insbesondere zur Behandlung von Neoplasien, Dermatosen und Altershaut.

## Patentansprüche (Patentansprüche für folgende(n) Vertragsstaat(en): ES)

1. Verfahren zur Herstellung von Verbindungen der allgemeinen Formel 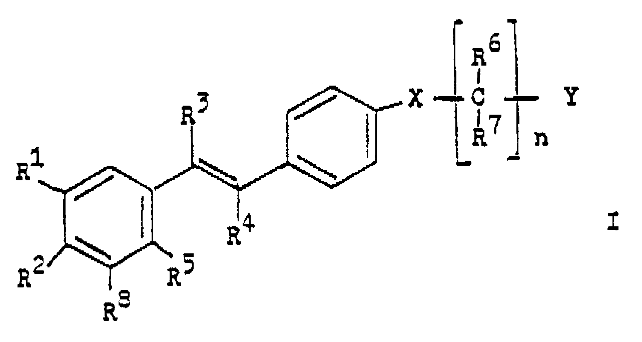 worin R¹ und R² unabhängig voneinander nieder Alkyl; oder zusammengenommen 1,3-Propylen, 1,4-Butylen und 1,5-Pentylen und nieder-Alkyl-substituierte Derivate davon; einer der Reste R³ und R⁴ Wasserstoff und der andere Wasserstoff oder nieder-Alkyl; R⁶ und R⁷ Wasserstoff oder nieder-Alkyl; R⁵ und R⁸ Wasserstoff, nieder-Alkyl, nieder-Alkoxy oder Halogen; X -O-; Y Piperidino, Pyrrolidino, Morpholino, Piperazino, N-Methylpiperazino, Thiomorpholino, Thiomorpholino-4-oxid, Thiomorpholino-4,4-dioxid, Imidazolino oder Pyrrolo und n 2, 3 oder 4 bedeutet, und wobei "nieder" Gruppen mit 1-6 C - Atomen bezeichnet,
**dadurch gekennzeichnet, dass** man
a) eine Verbindung der allgemeinen Formel 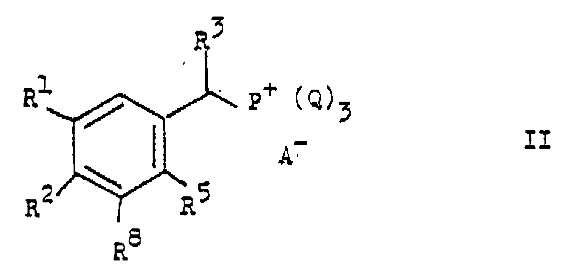 mit einer Verbindung der allgemeinen Formel 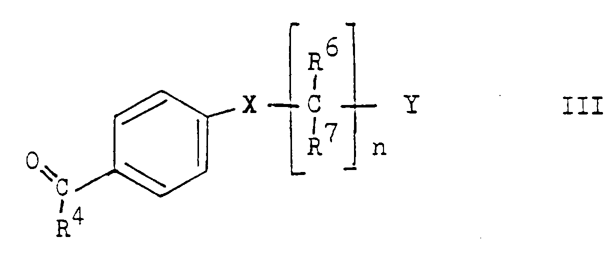 oder
b) eine Verbindung der allgemeinen Formel 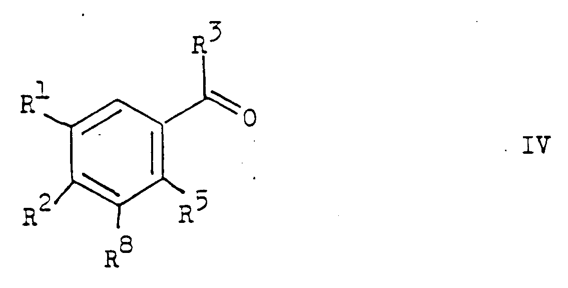 mit einer Verbindung der allgemeinen Formel 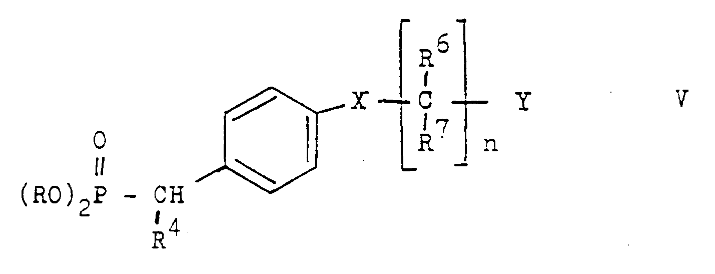 oder
c) eine Verbindung der allgemeinen Formel 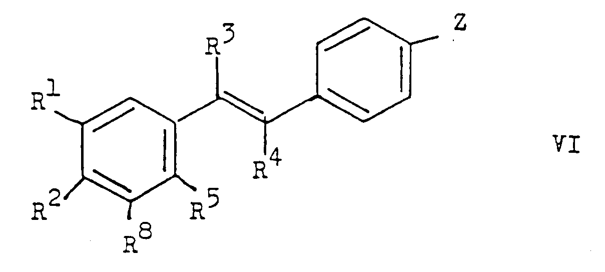 mit einer Verbindung der allgemeinen Formel 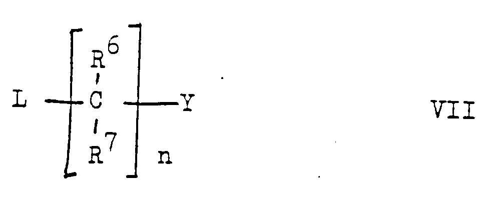 umsetzt, wobei in den vorgenannten Formeln II-VII R¹, R², R³, R⁴, R⁵, R⁶, R⁷, R⁸, X, Y und n die oben angegebene Bedeutung haben; Q Aryl; A⁻ ein Anion einer anorganischen oder organischen Säure; R eine niedere Alkylgruppe; Z Hydroxy; und L eine Abgangsgruppe darstellen.

2. Verfahren nach Anspruch 1 zur Herstellung von Verbindungen der allgemeinen Formel I, in denen R¹ und R² gemeinsam einen Rest -(CH₃)₂C-CH₂CH₂-C(CH₃)₂- oder -(CH₃)₂C-CH₂-C(CH₃)₂- darstellen.

3. Verfahren nach Anspruch 1 zur Herstellung von 4-[2-[p-[(E)-2-(5,6,7,8-Tetrahydro-5,5,8,8-tetramethyl-2-naphthyl)propenyl]phenoxy]äthyl]morpholin.

4. Verfahren nach Anspruch 1 zur Herstellung von 4-[2-[p-[(E)-2-(1,1,3,3-Tetramethyl-5-indenyl)propenyl]phenoxy]äthyl]morpholin.
1-[2-[p-[(E)-2-(5,6,7,8-Tetrahydro-5,5,8,8-tetramethyl-2-naphthyl)propenyl]phenoxy]äthyl]pyrrolidin.
1-[2-[p-[(E)-2-(5,6,7,8-Tetrahydro-5,5,8,8-tetramethyl-2-naphthyl)propenyl]phenoxy]äthyl]piperidin und
4-[2-[p-[(E)-2-(6,7,8,9-Tetrahydro-7,7-dimethyl-5H-benzocyclohepten-2-yl)propenyl]phenoxy]äthyl]morpholin.

5. Verfahren nach Anspruch 1 zur Herstellung von Tetrahydro-4-[2-[p-[(E)-2-(5,6,7,8-tetrahydro-5,5,8,8-tetramethyl-2-naphthyl)propenyl]phenoxy]äthyl]-2H-1,4-thiazin-1,1-dioxid.

6. Verwendung der Verbindungen gemäss den Ansprüchen 1-5 als Wirkstoffe bei der Herstellung pharmazeutischer Präparate, insbesondere zur Behandlung von Neoplasien, Dermatosen und Altershaut.

## Patentansprüche (Patentansprüche für folgende(n) Vertragsstaat(en): GR)

1. Verfahren zu Herstellung von Verbindungen der allgemeinen Formel 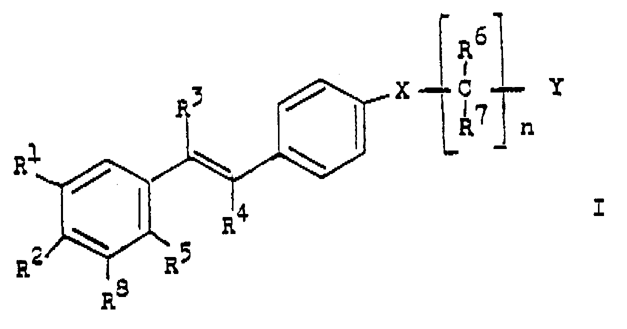 worin R¹ und R² unabhängig voneinander nieder Alkyl; oder zusammengenommen 1,3-Propylen, 1,4-Butylen und 1,5-Pentylen und nieder-Alkyl-substituierte Derivate davon; einer der Reste R³ und R⁴ Wasserstoff und der andere Wasserstoff oder nieder-Alkyl; R⁶ und R⁷ Wasserstoff oder nieder-Alkyl; R⁵ und R⁸ Wasserstoff, nieder-Alkyl, nieder-Alkoxy oder Halogen; X -O-; Y Piperidino, Pyrrolidino, Morpholino, Piperazino, N-Methylpiperazino, Thiomorpholino, Thiomorpholino-4-oxid, Thiomorpholino-4,4-dioxid, Imidazolino oder Pyrrolo und n 2, 3 oder 4 bedeutet,wobei "nieder" Gruppen mit 1-6 C - Atomen bezeichnet,
**dadurch gekennzeichnet, dass** man
a) eine Verbindung der allgemeinen Formel 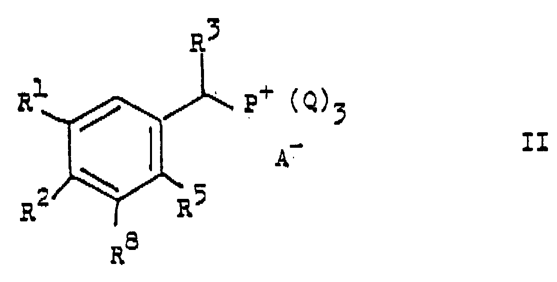 mit einer Verbindung der allgemeinen Formel 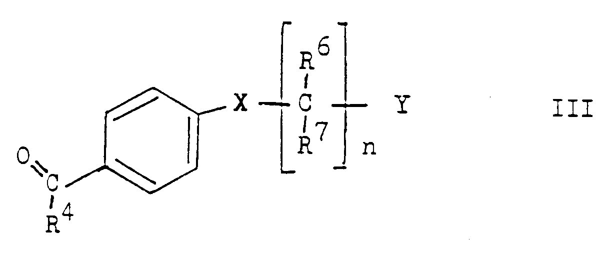 oder
b) eine Verbindung der allgemeinen Formel 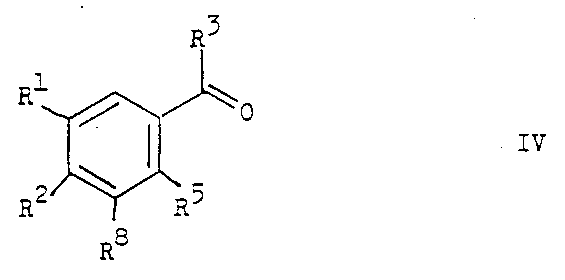 mit einer Verbindung der allgemeinen Formel 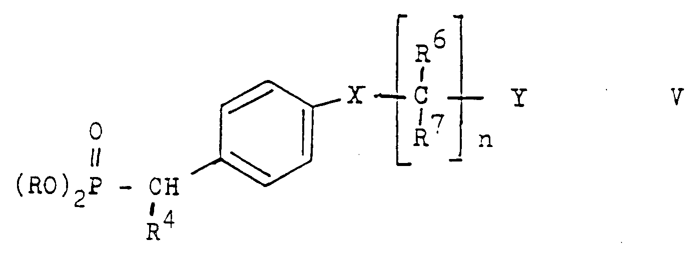 oder
c) eine Verbindung der allgemeinen Formel 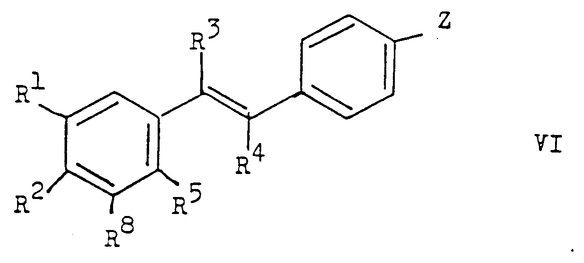 mit einer Verbindung der allgemeinen Formel 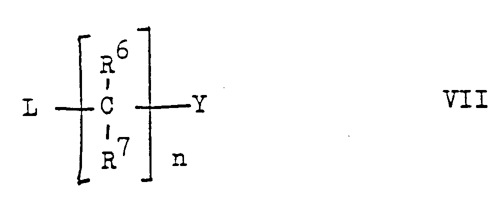 umsetzt, wobei in den vorgenannten Formeln II-VII R¹, R², R³, R⁴, R⁵, R⁶, R⁷, R⁸, X, Y und n die oben angegebene Bedeutung haben; Q Aryl; A⁻ ein Anion einer anorganischen oder organischen Säure; R eine niedere Alkylgruppe; Z Hydroxy; und L eine Abgangsgruppe darstellen.

2. Verfahren nach Anspruch 1 zur Herstellung von Verbindungen der allgemeinen Formel I, in denen R¹ und R² gemeinsam einen Rest -(CH₃)₂C-CH₂CH₂-C(CH₃)₂- oder -(CH₃)₂C-CH₂-C(CH₃)₂- darstellen.

3. Verfahren nach Anspruch 1 zur Herstellung von 4-[2-[p-[(E)-2-(5,6,7,8-Tetrahydro-5,5,8,8-tetramethyl-2-naphthyl)propenyl)phenoxy]äthyl]morpholin.

4. Verfahren nach Anspruch 1 zur Herstellung von 4-[2-[p-[(E)-2-(1,1,3,3-Tetramethyl-5-indenyl)propenyl]phenoxy]äthyl]morpholin.
1-[2-[p-[(E)-2-(5,6,7,8-Tetrahydro-5,5,8,8-tetramethyl-2-naphthyl)propenyl]phenoxy]äthyl]pyrrolidin,
1-[2-[p-[(E)-2-(5,6,7,8-Tetrahydro-5,5,8,8-tetramethyl-2-naphthyl)propenyl]phenoxy]äthyl]piperidin und
4-[2-[p-[(E)-2-(6,7,8,9-Tetrahydro-7,7-dimethyl-5H-benzocyclohepten-2-yl)propenyl]phenoxy]äthyl]morpholin.

5. Verfahren nach Anspruch 1 zur Herstellung von Tetrahydro-4-[2-[p-[(E)-2-(5,6,7,8-tetrahydro-5,5,8,8-tetramethyl-2-naphthyl)propenyl]phenoxy]äthyl]-2H-1,4-thiazin-1,1-dioxid.

6. Verwendung der Verbindungen gemäss den Ansprüchen 1-5 als Wirkstoffe bei der Herstellung pharmazeutischer Präparate, insbesondere zur Behandlung von Neoplasien, Dermatosen und Altershaut.

## Claims (Claims for the following Contracting State(s): AT, BE, CH, DE, FR, GB, IT, LI, LU, NL, SE)

1. Compounds of the general formula 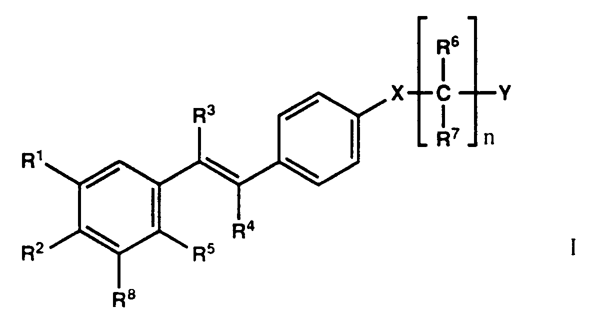 wherein R¹ and R² each independently represent lower-alkyl; or together represent 1,3-propylene, 1,4-butylene and 1,5-pentylene and lower-alkyl-substituted derivatives thereof; one of the residues R³ and R⁴ signifies hydrogen and the other signifies hydrogen or lower-alkyl; R⁶ and R⁷ signify hydrogen or lower-alkyl; R⁵ and R⁸ signify hydrogen, lower-alkyl, lower-alkoxy or halogen; X signifies -O-; Y signifies piperidino, pyrrolidino, morpholino, piperazino, N-methylpiperazino, thiomorpholino, thiomorpholino 4-oxide, thiomorpholino 4,4-dioxide, imidazolino or pyrrolo and n signifies 2, 3 or 4 and whereby "lower" denotes groups with 1-6 C atoms.

2. Compounds in accordance with claim 1, in which R¹ and R² together represent a residue -(CH₃)₂C-CH₂CH₂-C(CH₃)₂-or -(CH₃)₂C-CH₂-C(CH₃)₂-.

3. 4-[2-[p-[(E)-2-(5,6,7,8-Tetrahydro-5,5,8,8-tetramethyl-2-naphthyl)propenyl]phenoxy]ethyl]morpholine as a compound according to claim 1.

4. 4-[2-[p-[(E)-2-(1,1,3,3-Tetramethyl-5-indenyl)propenyl]phenoxy]ethyl]morpholine,
1-[2-[p-[(E)-2-(5,6,7,8-tetrahydro-5,5,8,8-tetramethyl-2-naphthyl)propenyl]phenoxy]ethyl]pyrrolidine,
1-[2-[p-[(E)-2-(5,6,7,8-tetrahydro-5,5,8,8-tetramethyl-2-naphthyl)propenyl]phenoxy]ethyl]piperidine,
4-[2-[p-[(E)-2-(6,7,8,9-tetrahydro-7,7-dimethyl-5H-benzocyclohepten-2-yl)propenyl]phenoxy]ethyl]morpholine,
tetrahydro-4-[2-[p-[(E)-2-(5,6,7,8-tetrahydro-5,5,8,8-tetramethyl-2-naphthyl)propenyl]phenoxy]ethyl]-2H-1,4-thiazine 1,1-dioxide as compounds according to claim 1.

5. The compounds in accordance with claims 1-4 for use as medicaments.

6. A process for the manufacture of the compounds of general formula 1 of claim 1, characterized by
a) reacting a compound of the general formula 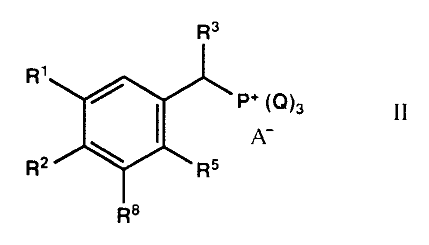 with a compound of the general formula 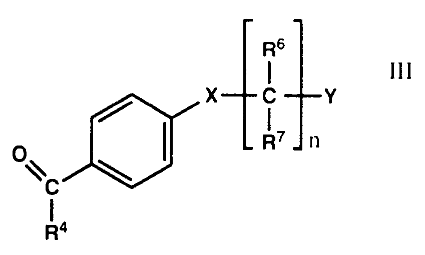 or
b) reacting a compound of the general formula 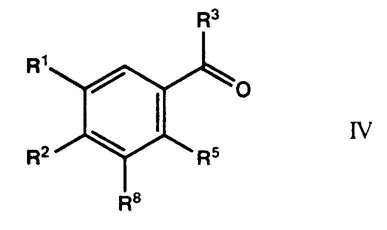 with a compound of the general formula 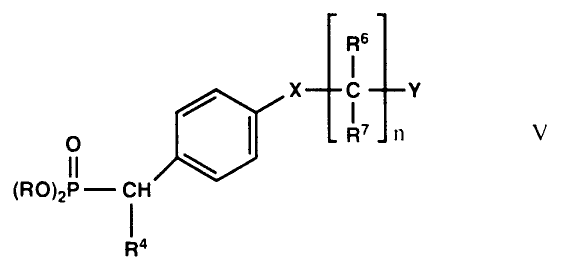 or
c) reacting a compound of the general formula 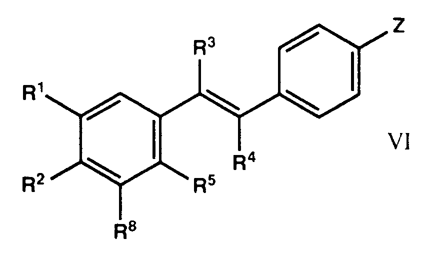 with a compound of the general formula 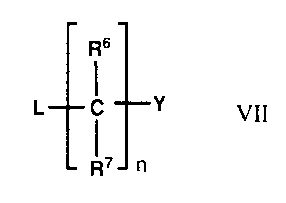 wherein in the foregoing formulae II-VII R¹, R², R³, R⁴, R⁵, R⁶, R⁷, R⁸, X, Y and n have the significance given above; Q represents aryl; A⁻ represents an anion of an inorganic or organic acid; R represents a lower alkyl group; Z represents hydroxy, and L represents a leaving group.

7. Pharmaceutical preparations containing a compound in accordance with claims 1-4 and usual pharmaceutical adjuvant and carrier materials.

8. The use of the compounds in accordance with claims 1-4 as active substances in the manufacture of pharmaceutical preparations, especially for the treatment of neoplasms, dermatoses and ageing of the skin.

## Claims (Claims for the following Contracting State(s): ES)

1. A process for the manufacture of compounds of the general formula 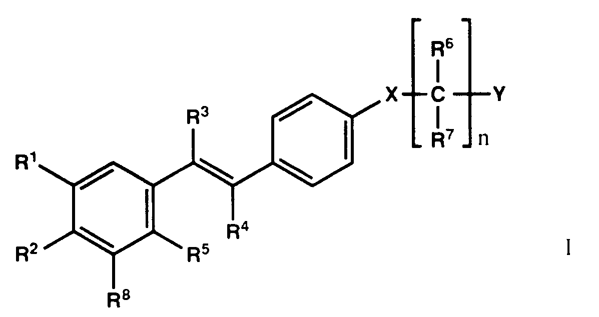 wherein R¹ and R² each independently represent lower-alkyl; or together represent 1,3-propylene, 1,4-butylene and 1,5-pentylene and lower-alkyl-substituted derivatives thereof; one of the residues R³ and R⁴ signifies hydrogen and the other signifies hydrogen or lower-alkyl; R⁶ and R⁷ signify hydrogen or lower-alkyl; R⁵ and R⁸ signify hydrogen, lower-alkyl, lower-alkoxy or halogen; X signifies -O-; Y signifies piperidino, pyrrolidino, morpholino, piperazino, N-methylpiperazino, thiomorpholino, thiomorpholino 4-oxide, thiomorpholino 4,4-dioxide, imidazolino or pyrrolo and n signifies 2, 3 or 4, whereby "lower" denotes groups with 1-6 C atoms,
characterized by
a) reacting a compound of the general formula 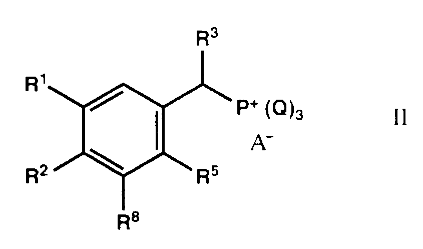 with a compound of the general formula 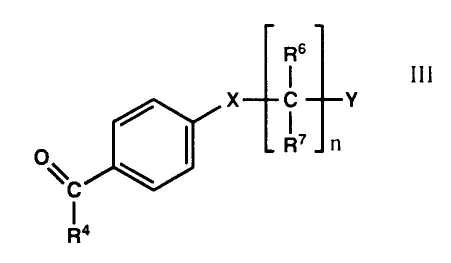 or
b) reacting a compound of the general formula 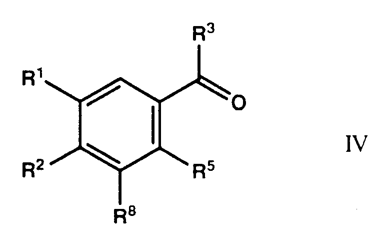 with a compound of the general formula 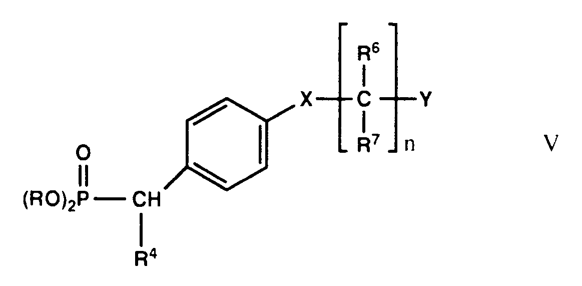 or
c) reacting a compound of the general formula 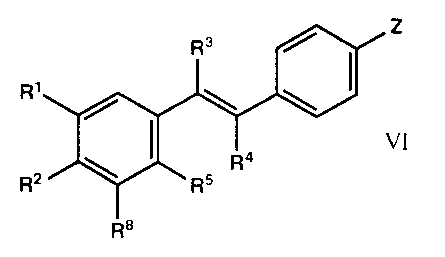 with a compound of the general formula 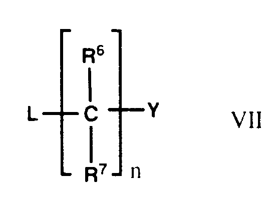 wherein in the foregoing formulae II-VII R¹, R², R³, R⁴, R⁵, R⁶, R⁷, R⁸, X, Y and n have the significance given above; Q represents aryl; A⁻ represents an anion of an inorganic or organic acid; R represents a lower alkyl group; Z represents hydroxy; and L represents a leaving group.

2. A process in accordance with claim 1 for the manufacture of compounds of formula I in which R¹ and R² together represent a residue -(CH₃)₂C-CH₂CH₂-C(CH₃)₂- or -(CH₃)₂C-CH₂-C(CH₃)₂-.

3. A process according to claim 1 for the manufacture of 4-[2-[p-[(E)-2-(5,6,7,8-tetrahydro-5,5,8,8-tetramethyl-2-napthyl)propenyl]phenoxy]ethyl]morpholine.

4. A process according to claim 1 for the manufacture of 4-[2-[p-[(E)-2-(1,1,3,3-tetramethyl-5-indenyl)-propenyl]phenoxy]ethyl]morpholine,
1-[2-(p-[(E)-2-(5,6,7,8-tetrahydro-5,5,8,8-tetramethyl-2-naphthyl)propenyl]phenoxy]ethyl]pyrrolidine,
1-[2-[p-[(E)-2-(5,6,7,8-tetrahydro-5,5,8,8-tetramethyl-2-naphthyl)propenyl]phenoxy]ethyl]piperidine and
4-[2-[p-[(E)-2-(6,7,8,9-tetrahydro-7,7-dimethyl-5H-benzocyclohepten-2-yl)propenyl]phenoxy]ethyl]morpholine.

5. A process according to claim 1 for the manufacture of tetrahydro-4-[2-[p-[(E)-2-(5,6,7,8-tetrahydro-5,5,8,8-tetramethyl-2-naphthyl)propenyl]phenoxy]ethyl]-2H-1,4-thiazine 1,1-dioxide.

6. The use of the compounds in accordance with claims 1-5 as active substances in the manufacture of pharmaceutical preparations, especially for the treatment of neoplasms, dermatoses and ageing of the skin.

## Claims (Claims for the following Contracting State(s): GR)

1. A process for the manufacture of compounds of the general formula 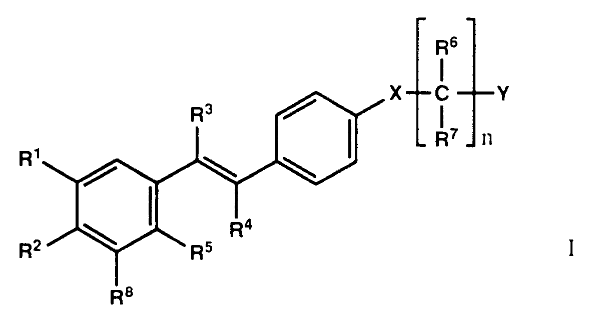 wherein R¹ and R² each independently represent lower-alkyl; or together represent 1,3-propylene, 1,4-butylene and 1,5-pentylene and lower-alkyl-substituted derivatives thereof; one of the residues R³ and R⁴ signifies hydrogen and the other signifies hydrogen or lower-alkyl; R⁶ and R⁷ signify hydrogen or lower-alkyl; R⁵ and R⁸ signify hydrogen, lower-alkyl, lower-alkoxy or halogen; X signifies -O-; Y signifies piperidino, pyrrolidino, morpholino, piperazino, N-methylpiperazino, thiomorpholino, thiomorpholino 4-oxide, thiomorpholino 4,4-dioxide, imidazolino or pyrrolo and n signifies 2, 3 or 4, whereby "lower" denotes groups with 1-6 C atoms,
characterized by
a) reacting a compound of the general formula 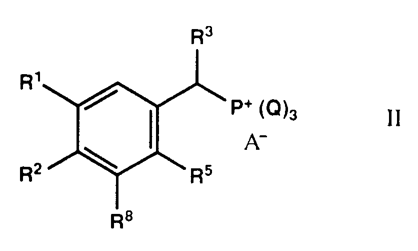 with a compound of the general formula 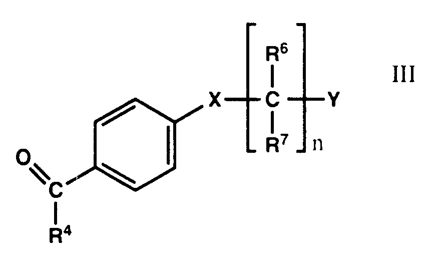 or
b) reacting a compound of the general formula 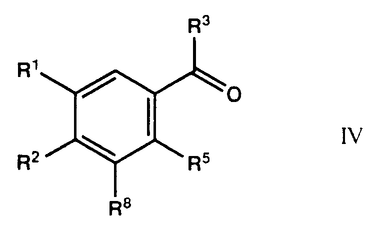 with a compound of the general formula 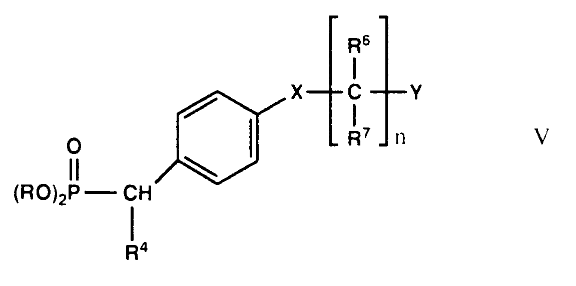 or
c) reacting a compound of the general formula 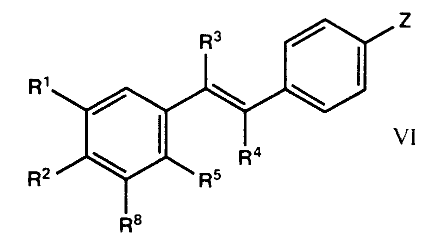 with a compound of the general formula 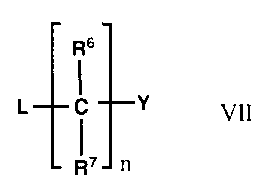 wherein in the foregoing formulae II-VII R¹, R², R³, R⁴, R⁵, R⁶, R⁷, R⁸, X, Y and n have the significance given above; Q represents aryl; A⁻ represents an anion of an inorganic or organic acid; R represents a lower alkyl group; Z represents hydroxy; and L represents a leaving group.

2. A process in accordance with claim 1 for the manufacture of compounds of formula I in which R¹ and R² together represent a residue -(CH₃)₂C-CH₂CH₂-C(CH₃)₂- or -(CH₃)₂C-CH₂-C(CH₃)₂-.

3. A process according to claim 1 for the manufacture of 4-[2-[p-[(E)-2-(5,6,7,8-tetrahydro-5,5,8,8-tetramethyl-2-naphthyl)propenyl]phenoxy]ethyl]morpholine.

4. A process according to claim 1 for the manufacture of 4-[2-[p-[(E)-2-(1,1,3,3-tetramethyl-5-indenyl)propenyl]phenoxy]ethyl]morpholine,
1-[2-[p-[(E)-2-(5,6,7,8-tetrahydro-5,5,8,8-tetramethyl-2-naphthyl)propenyl]phenoxy]ethyl]pyrrolidine,
1-[2-[p-[(E)-2-(5,6,7,8-tetrahydro-5,5,8,8-tetramethyl-2-naphthyl)propenyl]phenoxy]ethyl]piperidine and
4-[2-[p-[(E)-2-(6,7,8,9-tetrahydro-7,7-dimethyl-5H-benzocyclohepten-2-yl)propenyl]phenoxy]ethyl]morpholine.

5. A process according to claim 1 for the manufacture of tetrahydro-4-[2-[p-[(E)-2-(5,6,7,8-tetrahydro-5,5,8,8-tetramethyl-2-naphthyl)propenyl]phenoxy]ethyl]-2H-1,4-thiazine 1,1-dioxide.

6. The use of the compounds in accordance with claims 1-5 as active substances in the manufacture of pharmaceutical preparations, especially for the treatment of neoplasms, dermatoses and ageing of the skin.

## Revendications (Revendications pour l'(les) Etat(s) contractant(s) suivant(s): AT, BE, CH, DE, FR, GB, IT, LI, LU, NL, SE)

1. Composés de formule générale : 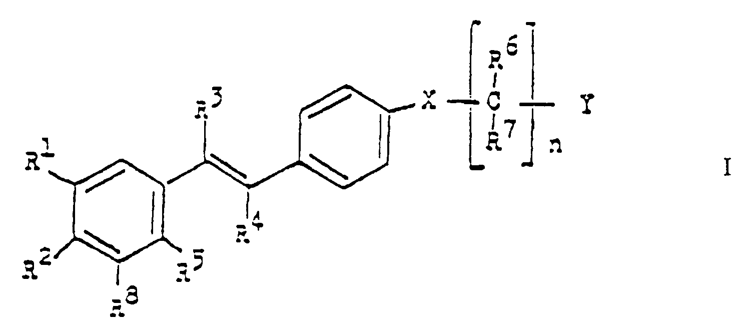 où R¹ et R² représentent, indépendamment l'un de l'autre, un alkyle inférieur ; ou pris ensemble, le 1,3-propylène, le 1,4-butylène et 1,5-pentylène et leurs dérivés substitués par un alkyle inférieur; l'un des restes R³ et R⁴ représente l'hydrogène et l'autre reste l'hydrogène ou un alkyle inférieur ; R⁶ et R⁷ représentent l'hydrogène ou un alkyle inférieur; R⁵ et R⁸ représentent l'hydrogène, un alkyle inférieur, un aloxy inférieur ou un halogène; X représente -O-; Y le pipéridino, le pyrrolidino, le morpholino, le pipérazino, le N-méthylpipérazino, le thiomorpholino, le thiomorpholino-4-oxyde, le thiomorpholino-4,4-dioxyde, l'imidazolino ou le pyrrolo et n est égal à 2, 3 ou 4, le terme "inférieur" désignant des groupes ayant 1 à 6 atomes de C.

2. Composés selon la revendication 1 dans lesquels R1 et R² représentent ensemble un reste -(CH₃)₂C-CH₂CH₂-C(CH₃)₂- ou -(CH₃)₂C-CH₂-C(CH₃)₂-.

3. La 4-[2-[p-[(E)-2-(5,6,7,8-tétrahydro-5,5,8,8-tétraméthyl-2-naphtyl)propényl]phénoxy] éthyl]-morpholine comme composé selon la revendication 1.

4. La 4-[2-[p-[(E)-2-(1,1,3,3-tétraméthyl-5-indényl)-propényl]-phénoxy]éthyl]morpholine,
la 1-[2-[p-[(E)-2-(5,6,7,8-tétrahydro-5,5,8,8-tétraméthyl-2-naphtyl)-propényl]phénoxy]-éthyl]-pyrrolidine
la 1-[2-[p-[(E)-2-(5,6,7,8-tétrahydro-5,5,8,8-tétraméthyl-2-naphtyl)-propényl] phénoxy]-éthyl]- pipéridine,
la 4-[2-[p-[(E)-2-(6,7,8,9-tétrahydro-7,7-diméthyl-5H-benzocycloheptén-2-yl)propényl]-phénoxy]-éthyl]-morpholine,
le tétrahydro-4-[2-[p-[(E)-2-(5,6,7,8-tétrahydro-5,5,8,8-tétraméthyl-2-naphtyl)propényl]-phénoxy]-éthyl]-2H-1,4-thiazine-1,1-dioxyde,
comme composés selon la revendication 1.

5. Composés selon les revendications 1 à 4 destinés à être utilisés comme médicaments.

6. Procédé pour la préparation des composés de formule générale I selon la revendication 1, caractérisé
a) en ce que l'on fait réagir un composé de formule générale 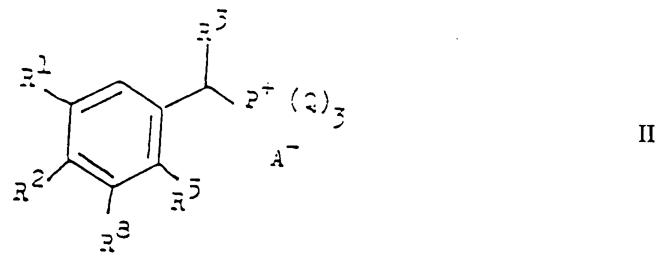 avec un composé de formule générale 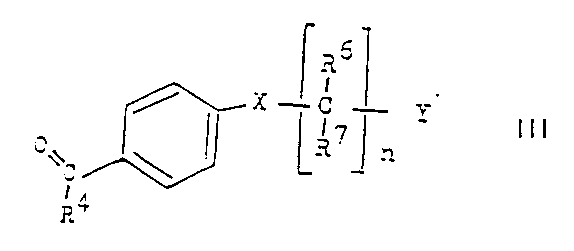 ou
b) en ce que l'on fait réagir un composé de formule générale 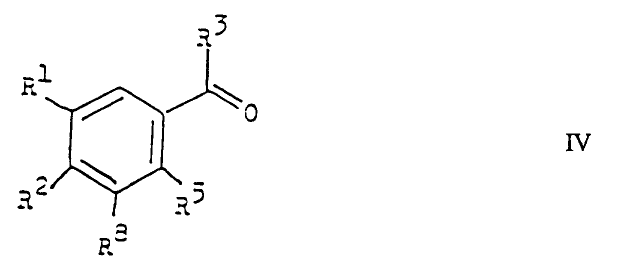 avec un composé de formule générale 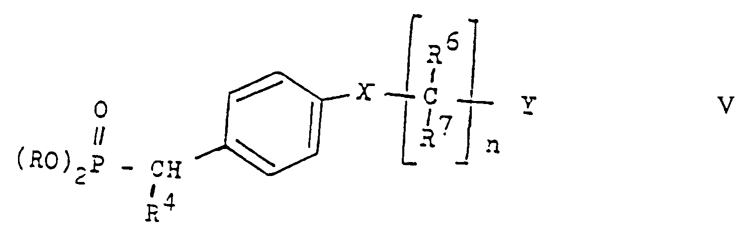 ou
c) en ce que l'on fait réagir un composé de formule générale 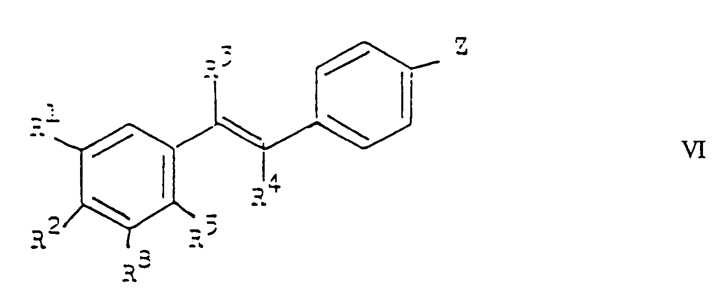 avec un composé de formule générale 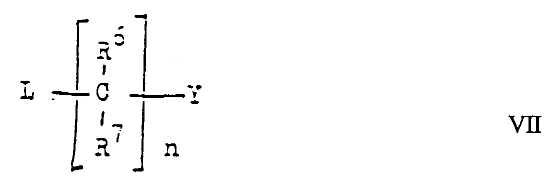 où dans les formules II à VII précitées R¹, R², R³, R⁴, R⁵, R⁶, R⁷, R⁸, X, Y et n ont les significations données ci-dessus; Q représente un aryle ; A⁻ représente un anion d'un acide minéral ou organique ; R un groupe alkyle inférieur ; Z un hydroxy; et L un groupe partant.

7. Préparations pharmaceutiques contenant un composé selon les revendications 1 à 4 et des adjuvants et supports pharmaceutiques usuels.

8. Utilisation des composés selon les revendications 1 à 4 comme principes actifs pour la préparation de compositions pharmaceutiques, en particulier pour le traitement des néoplasies, dermatoses et gérodermie.

## Revendications (Revendications pour l'(les) Etat(s) contractant(s) suivant(s): ES)

1. Procédé pour la préparation des composés de formule générale : 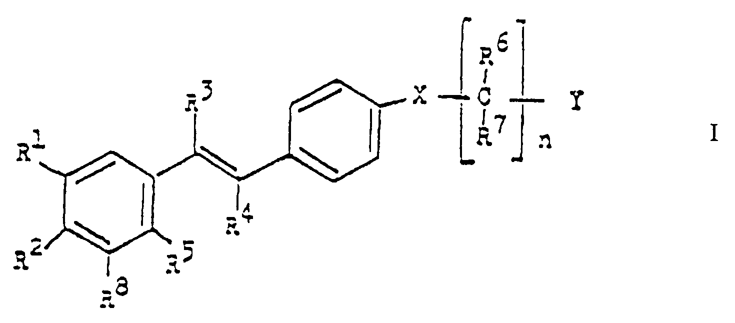 où R¹ et R² représentent, indépendamment l'un de l'autre, un alkyle inférieur ; ou pris ensemble, le 1,3-propylène, le 1,4-butylène et 1,5-pentylène et leurs dérivés substitués par un alkyle inférieur ; l'un des restes R³ et R⁴ représente l'hydrogène et l'autre reste l'hydrogène ou un alkyle inférieur ; R⁶ et R⁷ représentent l'hydrogène ou un alkyle inférieur; R⁵ et R⁸ représentent l'hydrogène, un alkyle inférieur, un aloxy inférieur ou un halogène ; X représente -O-; Y le pipéridino, le pyrrolidino, le morpholino, le pipérazino, le N-méthylpipérazino, le thiomorpholino, le thiomorpholino-4-oxyde, le thiomorpholino-4,4-dioxyde, l'imidazolino ou le pyrrolo et n est égal à 2, 3 ou 4, le terme "inférieur" désignant des groupes ayant 1 à 6 atomes de C, caractérisé
a) en ce que l'on fait réagir un composé de formule générale : 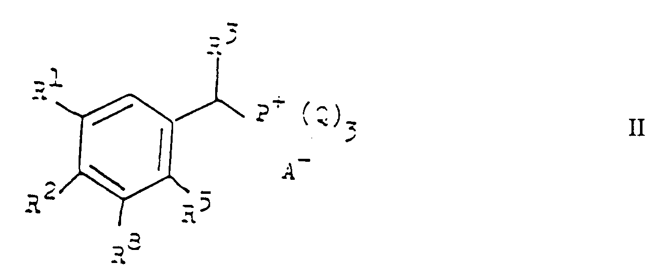 avec un composé de formule générale : 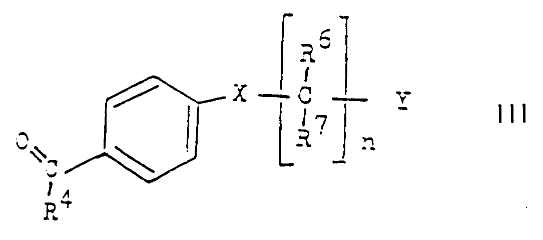 ou
b) en ce que l'on fait réagir un composé de formule générale : 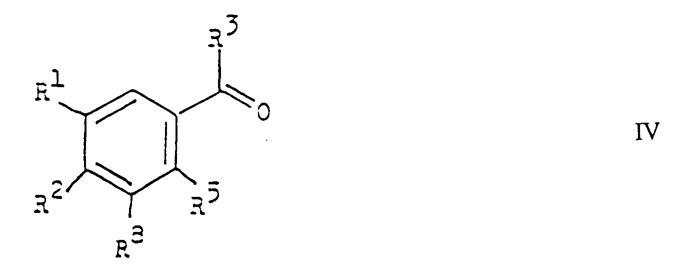 avec un composé de formule générale : 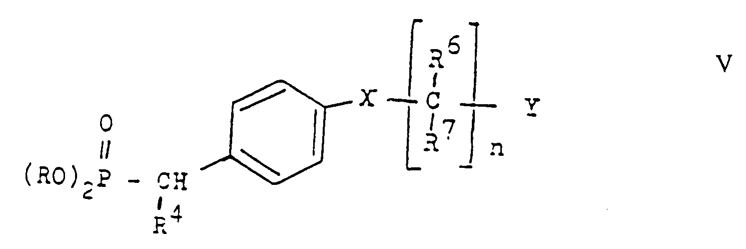 ou
c) en ce que l'on fait réagir un composé de formule générale : 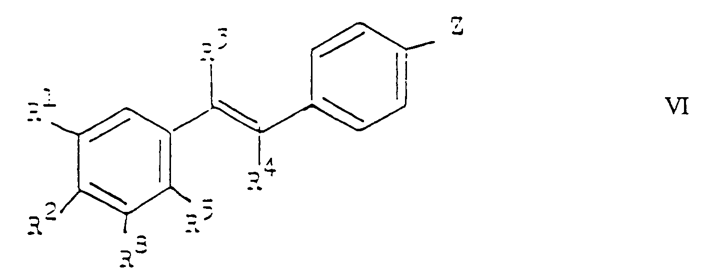 avec un composé de formule générale 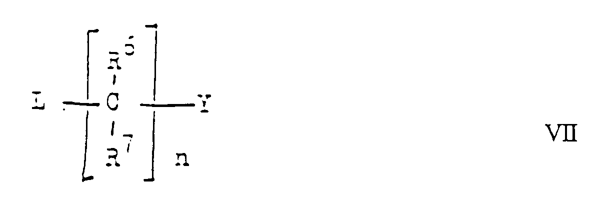 où dans les formules II à VII précitées R¹, R², R³, R⁴, R⁵, R⁶, R⁷, R⁸, X, Y et n ont les significations données ci-dessus; Q représente un aryle ; A⁻ représente un anion d'un acide minéral ou organique ; R un groupe alkyle inférieur ; Z un hydroxy; et L un groupe partant.

2. Procédé selon la revendication 1 pour la préparation des composés de formule générale I dans lesquels R¹ et R² représentent ensemble un reste -(CH₃)₂C-CH₂CH₂-C(CH₃)₂- ou -(CH₃)₂C-CH₂-C(CH₃)₂-.

3. Procédé selon la revendication 1 pour la préparation de la 4-[2-[p-[(E)-2-(5,6,7,8-tétrahydro-5,5,8,8-tétraméthyl-2-naphtyl)-propényl]-phénoxy]-éthyl]morpholine.

4. Procédé selon la revendication 1 pour la préparation de la 4-[2-[p-[(E)-2-(1,1,3,3-tétraméthyl-5-indényl)-propényl]-phénoxy]-ethyl]morpholine,
de la 1-[2-[p-[(E)-2-(5,6,7,8-tétrahydro-5,5,8,8-tétraméthyl-2-naphtyl)-propényl]phénoxy]-éthyl]-pyrrolidine
de la 1-[2-[p-[(E)-2-(5,6,7,8-tétrahydro-5,5,8,8-tétraméthyl-2-naphtyl)-propényl] phénoxy]-éthyl]- pipéridine, et
de la 4-[2-[p-[(E)-2-(6,7,8,9-tétrahydro-7,7-diméthyl-5H-benzocycloheptén-2-yl)propényl]-phénoxy]-éthyl]-morpholine,

5. Procédé selon la revendication 1 pour la préparation du tétrahydro-4-[2-[p-[(E)-2-(5,6,7,8-tétrahydro-5,5,8,8-tétraméthyl-2-naphtyl)-propényl]-phénoxy]-éthyl]-2H-1,4-thiazine-1,1-dioxyde.

6. Utilisation des composés selon les revendications 1 à 5 comme principes actifs pour la préparation de compositions pharmaceutiques, en particulier pour le traitement des néoplasies, dermatoses et gérodermie.

## Revendications (Revendications pour l'(les) Etat(s) contractant(s) suivant(s): GR)

1. Procédé pour la préparation des composés de formule générale : 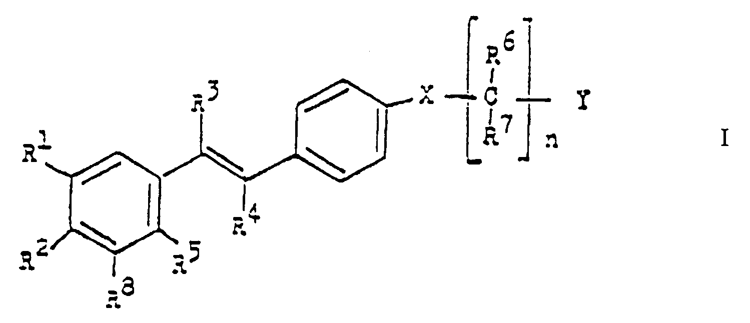 où R¹ et R² représentent, indépendamment l'un de l'autre, un alkyle inférieur ; ou pris ensemble, le 1,3-propylène, le 1,4-butylène et 1,5-pentylène et leurs dérivés substitués par un alkyle inférieur ; l'un des restes R³ et R⁴ représente l'hydrogène et l'autre reste l'hydrogène ou un alkyle inférieur ; R⁶ et R⁷ représentent l'hydrogène ou un alkyle inférieur; R⁵ et R⁸ représentent l'hydrogène, un alkyle inférieur, un aloxy inférieur ou un halogène ; X représente -O-; Y le pipéridino, le pyrrolidino, le morpholino, le pipérazino, le N-méthylpipérazino, le thiomorpholino, le thiomorpholino-4-oxyde, le thiomorpholino-4,4-dioxyde, l'imidazolino ou le pyrrolo et n est égal à 2, 3 ou 4, le terme "inférieur" désignant des groupes ayant 1 à 6 atomes de C, caractérisé
a) en ce que l'on fait réagir un composé de formule générale 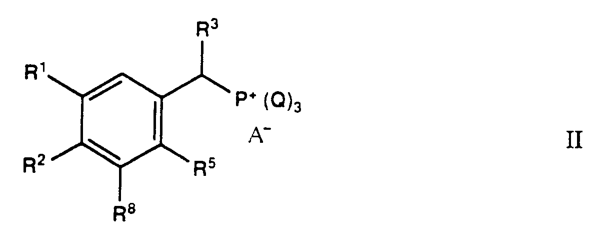 avec un composé de formule générale 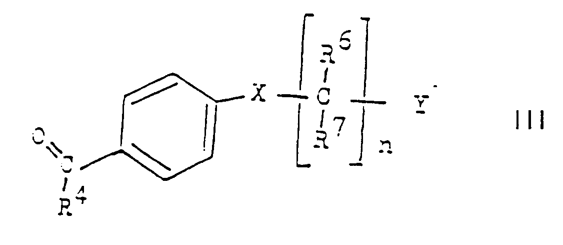 ou
b) en ce que l'on fait réagir un composé de formule générale 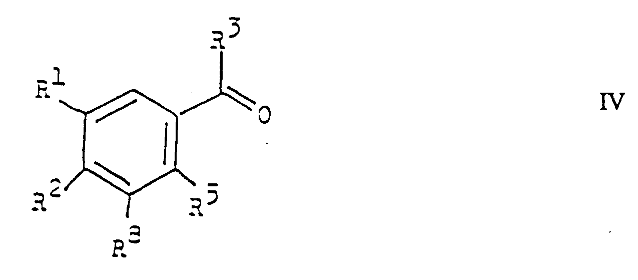 avec un composé de formule générale 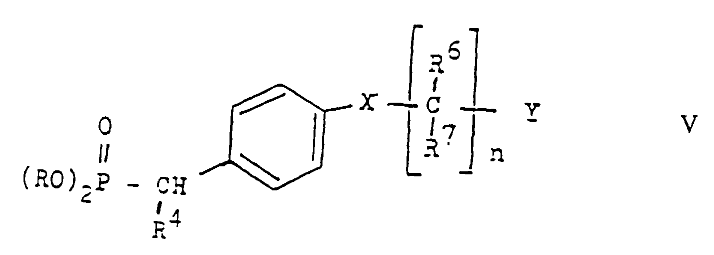 ou
c) en ce que l'on fait réagir un composé de formule générale 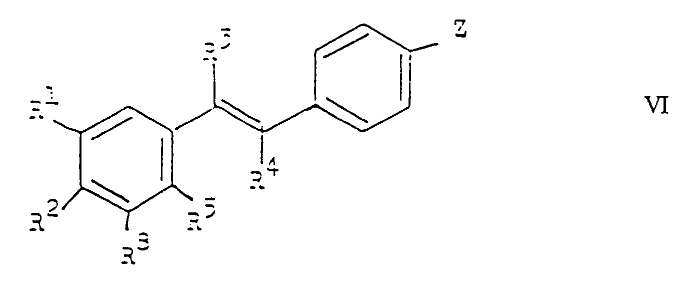 avec un composé de formule générale 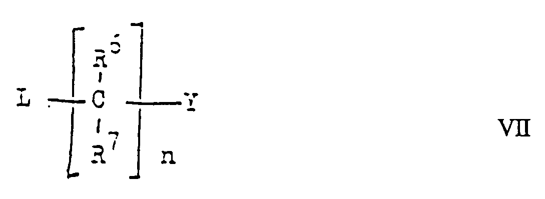 où dans les formules II à VII précitées R¹, R², R³, R⁴, R⁵, R⁶, R⁷, R⁸, X, Y et n ont les significations données ci-dessus; Q représente un aryle ; A⁻ représente un anion d'un acide minéral ou organique ; R un groupe alkyle inférieur ; Z un hydroxy; et L un groupe partant.

2. Procédé selon la revendication 1 pour la préparation des composés de formule générale I dans lesquels R¹ et R² représentent ensemble un reste -(CH₃)₂C-CH₂CH₂-C(CH₃)₂- ou -(CH₃)₂C-CH₂-C(CH₃)₂-.

3. Procédé selon la revendication 1 pour la préparation de la 4-[2-[p-[(E)-2-(5,6,7,8-tétrahydro-5,5,8,8-tétraméthyl-2-naphtyl)-propényl]-phénoxy]-éthyl]morpholine.

4. Procédé selon la revendication 1 pour la préparation de la
4-[2-[p-[(E)-2-(1,1,3,3-tétraméthyl-5-indényl)-propényl]-phénoxy]-éthyl]morpholine,
de la 1-[2-[p-[(E)-2-(5,6,7,8-tétrahydro-5,5,8,8-tétraméthyl-2-naphtyl)-propényl]phénoxy]-éthyl]-pyrrolidine
de la 1-[2-[p-[(E)-2-(5,6,7,8-tétrahydro-5,5,8,8-tétraméthyl-2-naphtyl)-propényl] phénoxy]-éthyl]-pipéridine, et
de la 4-[2-[p-[(E)-2-(6,7,8,9-tétrahydro-7,7-diméthyl-5H-benzocycloheptén-2-yl)propényl]-phénoxy]-éthyl]-morpholine,

5. Procédé selon la revendication 1 pour la préparation du tétrahydro-4-[2-[p-[(E)-2-(5,6,7,8-tétrahydro-5,5,8,8-tétraméthyl-2-naphtyl)-propényl]-phénoxy]-éthyl]-2H-1,4-thiazine-1,1-dioxyde.

6. Utilisation des composés selon les revendications 1 à 5 comme principes actifs pour la préparation de compositions pharmaceutiques, en particulier pour le traitement des néoplasies, dermatoses et gérodermie.
